# EUROPEAN PATENT APPLICATION

(11) **EP 2 067 442 A1**
(43) Date of publication of application: **10.06.2009**
(21) Application number: 08253854.7
(22) Date of filing: 01.12.2008
(51) Int. Cl.: A61B 17/16

(54) **Percutaneous devices for separating tissue and kit**

(30) Priority: 03.12.2007 US 991946 P; 22.11.2008 US 276326
(71) Applicant: Vertos Medical, Inc., San Jose, CA 95131 (US)
(72) Inventor: Sand, Paul M, San Carlos, CA (US)
(74) Representative: Price, Nigel John King

(57) **Abstract**

The disclosure describes devices for separating a first tissue from a second tissue at a surgical site, comprising, a hollow body having a distal end and a proximal end, wherein the distal end further comprises an upper separation member extendable laterally from hollow body at a first angle; an inner member slidably disposed within the hollow body, wherein the distal end of the inner member has a lower separation member extendable laterally from inner member at a second angle, wherein the upper separation member and the lower separation member have a first configuration and a second configuration into a tissue in the working zone on the first lateral side of the median plane. Additionally, methods for using the device and kits containing the device are disclosed.

## Description

### CROSS-REFERENCE

This application claims priority to U.S. Utility Application No. 12/276,326, filed November 22, 2008, and also claims the benefit of U.S. Provisional Application No. 60/991,946, filed December 3, 2007, which application is incorporated herein by reference.

### BACKGROUND

### Field of the Invention

The present invention relates generally to minimally invasive devices, systems methods and kits for treating spinal disorders; including devices, systems, methods and kits that use imaging guidance. More particularly, the present invention relates to devices, systems, methods and kits to reduce stenosis and increase the cross-sectional area of the spinal canal available for the spinal cord. Still more particularly, the present invention relates to devices, systems, methods and kits to percutaneously separate tissue such as portions of an enlarged ligamentum flavum from the lamina of a spine.

### Background of the Invention

Back pain is a common ailment. In many cases, the pain severely limits a person's functional ability and quality of life. Back pain interferes with work, routine daily activities, and recreation. It is estimated that Americans spend $50 billion each year on low back pain alone. It is the most common cause of job-related disability and a leading contributor to missed work. Spinal stenosis, a condition that results from narrowing of the spinal canal causing nerve pinching, leads to persistent pain in the buttocks, limping, lack of feeling in the lower extremities, and decreased physical activity. Spinal stenosis is considered a silent epidemic and occurs with an incidence of between 4% and 6% (or more) of adults aged 50 and older. It is also the most frequent reason cited for back surgery in patients aged 60 and older. Currently, it is estimated that as many as 400,000 Americans, most over the age of 60, may already be suffering from the symptoms of lumbar spinal stenosis according to The American Association of Neurological Surgeons (AANS) and The Congress of Neurological Surgeons (CNS). This number is expected to grow as members of the baby boom generation begin to reach their 60s over the next decade. Moreover, according to the U.S. Census Bureau, people over 60 will account for 18.7% of the domestic population in 2010 versus 16.6% in 1999.

Lumbar spinal stenosis is often defined as a dural sac cross-sectional area less than 100 mm² or an anterior-posterior (AP) dimension of the canal of less than 10-12 mm for an average male. The source of many cases of lumbar spinal stenosis is thickening of the ligamentum flavum. Spinal stenosis may also be caused by subluxation, facet joint hypertrophy, osteophyte formation, underdevelopment of spinal canal, spondylosis deformans, degenerative intervertebral discs, degenerative spondylolisthesis, degenerative arthritis, ossification of the vertebral accessory ligaments and the like. A less common cause of spinal stenosis, which usually affects patients with morbid obesity or patients on oral corticosteroids, is excess fat in the epidural space. The excessive epidural fat compresses the dural sac, nerve roots and blood vessels contained therein and resulting in back, leg pain and weakness and numbness of the legs. Spinal stenosis may also affect the cervical and, less commonly, the thoracic spine.

Patients suffering from spinal stenosis are typically first treated with a conservative approach. A more conservative approach is a combination of rest, support devices, physical therapy, and pain medications - including anti-inflammatory medications and epidural steroid injections. This treatment is normally given over the initial months after diagnosis in hope that it will correct the problem without requiring more drastic measures. When the pain/discomfort continues, a surgical procedure is discussed and pursued if the patient and their physician think it will improve the patient's quality of life. These conservative treatment options frequently fail. If symptoms are severe, surgery is required to decompress the spinal cord and nerve roots. Surgical options are invasive and include decompression or laminectomy, laminotomy, foramitomony and spinal fusion.

In some conventional surgical approaches to correct stenosis in the lumbar region, an incision is made in the back and the muscles and supporting structures are stripped away from the spine, exposing the posterior aspect of the vertebral column. The thickened ligamentum flavum is then exposed by removal of a portion of the vertebral arch, often at the laminae, covering the back of the spinal canal (laminectomy). The thickened ligamentum flavum ligament can then be excised by sharp dissection with a scalpel or punching instruments such as a Kerrison style punch that is used to remove small chips of tissue. The procedure is performed under general anesthesia. Patients are usually admitted to the hospital for approximately five to seven days depending on the age and overall condition of the patient. Patients usually require between six weeks and three months to recover from the procedure. Further, many patients need extended therapy at a rehabilitation facility to regain enough mobility to live independently. The risks associated with surgery include bleeding, blood clots and dural tears. In some cases surgical intervention fails to relieve symptoms, or the symptoms return over time.

Much of the pain and disability after an open laminectomy results from the tearing and cutting of the back muscles, blood vessels, supporting ligaments, and nerves that occurs during the exposure of the spinal column. Also, because the spine stabilizing back muscles and ligaments are stripped and detached from the spine during the laminectomy, these patients frequently develop spinal instability post-operatively.

Minimally invasive techniques offer an important potential for less post-operative pain and faster recovery compared to traditional open surgery. Percutaneous interventional spinal procedures can be performed with local anesthesia, thereby sparing the patient the risks and recovery time required with general anesthesia. In addition, there is less damage to the paraspinal muscles and ligaments with minimally invasive techniques, thereby reducing pain and preserving these important stabilizing structures.

Various techniques for minimally invasive treatment of the spine are known. Microdiscectomy is performed by making a small incision in the skin and deep tissues to create a portal to the spine. A microscope is then used to aid in the dissection of the adjacent structures prior to discectomy. The recovery for this procedure is much shorter than traditional open discectomies. Percutaneous discectomy devices with fluoroscopic guidance have been used successfully to treat disorders of the disc but not to treat spinal stenosis or the ligamentum flavum directly. Arthroscopy or direct visualization of the spinal structures using a catheter or optical system have also been proposed to treat disorders of the spine including spinal stenosis, however these devices still use miniaturized standard surgical instruments and direct visualization of the spine similar to open surgical procedures. These devices and techniques are limited by the small size of the canal and these operations are difficult to perform and master. In addition, these procedures are painful and often require general anesthesia. Further, the arthroscopy procedures are time consuming and the fiber optic systems are expensive to purchase and maintain.

Still further, because the nerves of the spinal cord pass through the spinal canal directly adjacent to and anterior to the ligamentum flavum, any surgery, regardless of whether open or percutaneous, includes a risk of damage to the nerves of the spinal cord.

Hence, it remains desirable to provide simple methods, techniques, and devices for treating spinal stenosis and other spinal disorders without requiring open surgery. It is further desired to provide a system whereby the risk of damage to the dural sac containing the spinal nerves may be reduced.

### SUMMARY OF THE INVENTION

Methods, devices and kits for separating ligament are described herein. Other aspects and features of the devices and methods will be described in more detail below. As will be appreciated by those skilled in the art, the devices and methods can be used in connection with a wide variety of tissue. However, for purposes of illustration, and without limitation, the devices and methods are described in the context of use within the spine.

An aspect of the invention is directed to devices for separating a first tissue from a second tissue at a surgical site. Suitable devices comprise: a hollow body having a distal end and a proximal end, wherein the distal end further comprises an upper separation member extendable laterally from the hollow body at a first angle; an inner member having a distal end and a proximal end and slidably disposed within the hollow body, wherein the distal end of the inner member has a lower separation member extendable laterally from the inner member at a second angle and wherein the upper separation member and the lower separation member have a first configuration and a second configuration. In some aspects, the hollow body can be adapted and configured to provide for a separation means that is laterally extendable from the hollow body. The devices can be configured such that the first angle is greater than 90 degrees. Additionally, the second angle can, in some cases, be the same as the first angle. In any configuration of these devices, the hollow body and the inner member can be configured such that they are substantially tubular. Moreover, the devices can be configured such that the upper separation member further comprises an outer face and an inner face and the lower separation member further comprises an outer face and an inner face and further wherein the separation members are constructed and arranged so that the inner face of the lower separation member is spaced between 3 and 16 mm from the inner face of the outer separation member in the first configuration. Separation members can be any suitable means for separating. Moreover, devices can also be configured to include a handle attachable to the proximal end of the hollow body. Furthermore, devices can be configured such that the upper separation member and the lower separation member have distal ends which are beveled. The upper separation member and the lower separation member can also be configured in any of the devices such that the members are angled to form a sharpened tip. In some aspects means for moving the upper separation member and the lower separation member between the open configuration and the closed configuration, the means being located at the proximal ends of the hollow body and the inner member. In still other aspects, an actuator can be provided that is coupled to the inner member. The actuator can, for example, be a motor or any other suitable mechanism or means for actuating. In some configurations of the devices, the motor is disposed within the handle. The devices disclosed herein are configured for use with connective tissue. In some aspects, the the first tissue is ligament and the second tissue is bone; in other aspects, the first tissue is a laminae of a vertebral body and the second tissue is a ligamentum flavum.

Another aspect of the invention is directed to a kit for tissue separation. Suitable kits comprise, for example, the devices disclosed herein and and packaging. Additional components of suitable kits include, but are not limited to, for example, one or more injectable media, such as a contrast medium; one or more hydrophillic-lipophillic block copolymer gels; one or more guides adaptable for use with the device.

Yet another aspect of the invention is directed to methods for treating stenosis in a spine of a patient with reference to a median plane of the patient. The methods comprise, for example, the steps of: compressing a dural sac in the region of interest by injecting a fluid to form a safety zone and establish a working zone in the region of interest, the safety zone lying between the working zone and the dural sac; percutaneously accessing an epidural space in the region of interest on a first lateral side of a median plane; and inserting a device for separating a first tissue from a second tissue at a surgical site, comprising, a hollow body having a distal end and a proximal end, wherein the distal end further comprises an upper separation member extendable laterally from hollow body at a first angle; an inner member having a distal end and a proximal end and slidably disposed within the hollow body, wherein the distal end of the inner member has a lower separation member extendable laterally from inner member at a second angle, wherein the upper separation member and the lower separation member have a first configuration and a second configuration into a tissue in the working zone on the first lateral side of the median plane. The methods can also include, where appropriate or clinically useful, the step of generating at least one view of a portion of a spinal canal in a region of interest. Where a view is obtained, at least one view can be used to position the tissue excision device during at least part of the step of inserting. In at least some methods, at least a portion of a patient's ligamentum flavum occupies the working zone in the region of interest. Moreover, the step of using the tissue separation device to percutaneously reduce a stenosis can be performed on the first lateral side of the median plane. Some steps can include using the at least one view to position the tissue excision device during at least part of the step of using the tissue excision device. In some aspects the steps can include removing at least a portion of the ligamentum flavum in the region of interest. Still other methods can include the step of using the tissue excision device to percutaneously reduce a stenosis on a second lateral side of the median plane different than the first lateral side. Furthermore, the steps can comprise using the at least one view to position the tissue excision device during at least part of the step of using the tissue excision device.

Another aspect of the invention is directed to the use of any of the devices disclosed herein to separate tissue and/or reducing stenosis in the spinal canal.

Embodiments described herein comprise a combination of features and advantages intended to address various shortcomings associated with certain prior devices. The various characteristics described above, as well as other features, will be readily apparent to those skilled in the art upon reading the following detailed description of the preferred embodiments, and by referring to the accompanying drawings.

### INCORPORATION BY REFERENCE

All publications, patents, and patent applications mentioned in this specification are herein incorporated by reference to the same extent as if each individual publication, patent, or patent application was specifically and individually indicated to be incorporated by reference.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:

**FIG. 1** is a lateral elevation view of a normal human spinal column;

**FIG. 2A** is a superior view of a normal human lumbar vertebra; **FIG. 2B** is a cross-section of the spine within the spinal canal with the dural sac and a normal (un-stenosed) ligamentum flavum therein;

**FIG. 3** is a lateral elevational view of two vertebral bodies forming a functional spinal unit;

**FIG. 4** is a posterolateral oblique view of a vertebrae from a human spinal column;

**FIG. 5** is a perspective view of the anatomical planes of the human body;

**FIGS. 6A-C** are side views of tissue separation devices including the distal end;

**FIG. 7** illustrates a tissue separation device of **FIG. 6A** with the operation features located within the device;

**FIGS. 8A-B** are cross-sectional views the distal end of an embodiment of a tissue separation device in a closed position and in an opened position;

**FIGS. 9A** and **9B** are side views of alternative embodiments for the separation members of FIGS. **8A** and **8B**;

**FIGS. 10A** and **10B** are top views of alternative embodiments for the separation members of **FIGS. 8A** and **8B****;**

**FIG. 11A** is an enlarged cross-section of a vertebral foramen, showing a safety zone created by compression of the dural sac; **FIG. 11B** is the cross-section **of** **FIG. 11A****,** showing a tissue separation tool positioned in the ligamentum flavum using an ipsilateral approach; **FIG. 11C** is the cross-section of **FIG. 11A****,** showing a tissue separation tool positioned in the ligamentum flavum using a minimally invasive decompression procedure;

**FIG. 12A** is a partial cross-section of the lumbar portion of the vertebral column; **FIGS. 12B-D** are the cross-sections of **FIG. 12A****,** showing the orientation of a tool relative to the vertebral column; **FIG. 12E** is the cross-section of **FIG. 12A****,** showing the orientation of an instrument relative to the vertebral column; and

**FIGS. 13A** and **13B** are side schematic views illustrating the device of **FIG. 6** separating a ligamentum flavum from a vertebral lamina.

### DETAILED DESCRIPTION OF THE PREFERRED EMBODIMENTS

The invention relates generally to devices, apparatus or mechanisms that are suitable for use within a human body to restore, modify and/or augment tissue, and systems therefor. As will be appreciated by those skilled in the art, tissue is an aggregation of morphologically similar cells and associated intercellular matter acting together to perform one or more specific functions in the body. There are four basic types of tissue: muscle, nerve, epidermal, and connective (which includes bone and ligament).

For purposes of illustrating the usefulness of the invention, the invention is described in the context of treating spinal pathologies. However, persons of skill in the art will appreciate that the devices can be used in conjunction with other pathologies without departing from the scope of the invention. In some instances the devices can include devices designed to separate body parts or structure. The devices, apparatus or mechanisms are configured such that the devices can be formed from parts, elements or components which alone or in combination comprise the device. The devices can also be configured such that one or more elements or components are formed integrally to achieve a desired physiological, operational or functional result such that the components complete the device. Functional results can include the surgical restoration and functional power of a patient, controlling, limiting or altering the functional power of a patient, and/or eliminating the functional power of a patient by preventing joint motion. Portions of the device can be configured to replace or augment existing anatomy and/or implanted devices, and/or be used in combination with resection or removal of existing anatomical structure.

### I. ANATOMICAL REVIEW

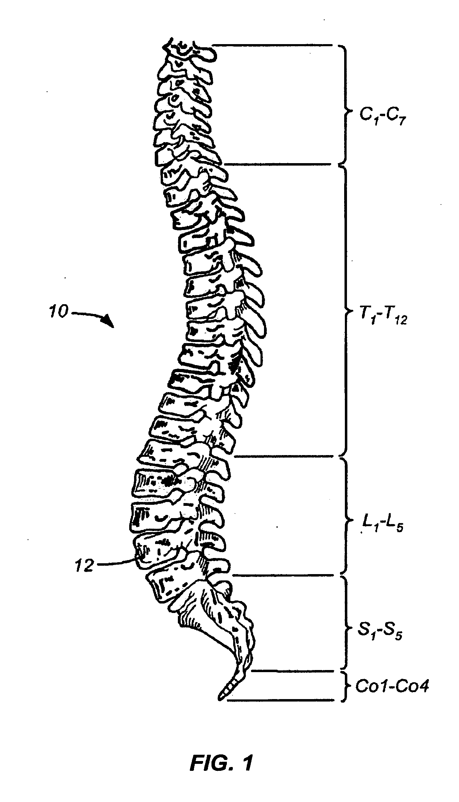

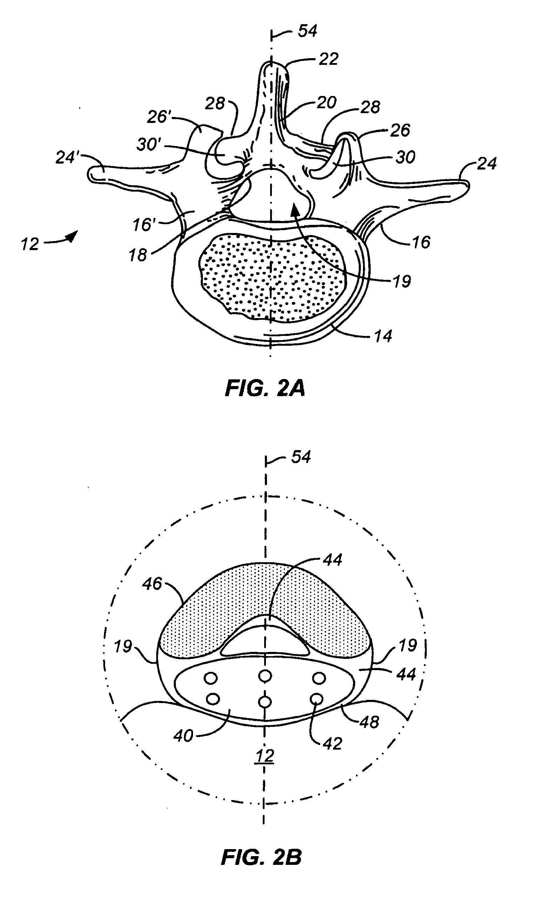

As discussed above, the devices and their usefulness can be illustrated in the context of spinal pathologies. In order to appreciate the usefulness of the devices it is helpful to understand an anatomical environment where the devices can be used. Thus, for example, the devices are designed to interact with the human spinal column ***10*,** as shown in **FIG. 1****,** which is comprised of a series of thirty-three stacked vertebrae ***12*** divided into five regions. The cervical region includes seven vertebrae, known as **C1-C7.** The thoracic region includes twelve vertebrae, known as **T1-T12.** The lumbar region contains five vertebrae, known as **L1-L5.** The sacral region is comprised of five fused vertebrae, known as **S1-S5,** while the coccygeal region contains four fused vertebrae, known as **Col-Co4.** An example of one of the vertebra is illustrated in **FIG. 2A** which depicts a superior plan view of a normal human lumbar vertebra ***12*.** Although human lumbar vertebrae vary somewhat according to location, the vertebrae share many common features. Each vertebra ***12*** includes a vertebral body ***14*.** Two short boney protrusions, the pedicles ***16, 16',*** extend dorsally from each side of the vertebral body ***14*** to form a vertebral arch ***18*** which defines the vertebral foramen ***19*** which houses the spinal cord and associated meninges. At the posterior end of each pedicle ***16*,** the vertebral arch ***18*** flares out into broad plates of bone known as the laminae ***20*.** The laminae ***20*** fuse with each other to form a spinous process ***22*.** The spinous process ***22*** provides for muscle and ligamentous attachment. A smooth transition from the pedicles ***16*** to the laminae ***20*** is interrupted by the formation of a series of processes.

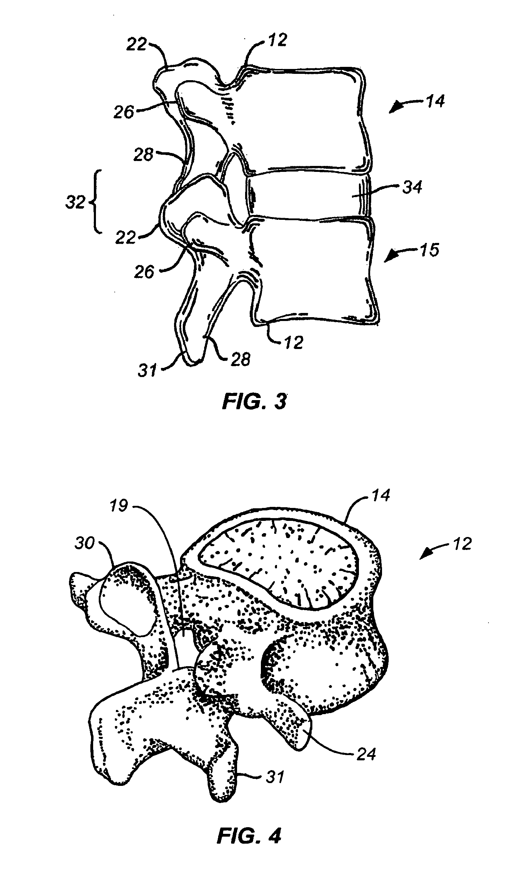

Two transverse processes ***24, 24*** thrust out laterally, one on each side, from the junction of the pedicle ***16*** with the lamina ***20*.** The transverse processes ***24, 24'*** serve as levers for the attachment of muscles to the vertebrae ***12*.** Four articular processes, two superior ***26, 26'*** and two inferior ***28, 28'*,** also rise from the junctions of the pedicles ***16*** and the laminae ***20*.** The superior articular processes ***26, 26'*** are sharp oval plates of bone rising upward on each side of the vertebrae, while the inferior processes ***28*, *28*'** are oval plates of bone that jut downward on each side. *See also* **FIG. 4****.**

The superior and inferior articular processes ***26*** and ***28*** each have a natural bony structure known as a facet. The superior articular facet ***30*** faces medially upward, while the inferior articular facet ***31*** (see **FIG. 3**) faces laterally downward. When adjacent vertebrae ***12*** are aligned, the facets ***30, 31,*** which are capped with a smooth articular cartilage and encapsulated by ligaments, interlock to form a facet joint ***32*.**

An intervertebral disc ***34*** located between each adjacent vertebra ***12*** (with stacked vertebral bodies shown as ***14, 15*** in **FIG. 3**) permits gliding movement between the vertebrae ***12*.** The structure and alignment of the vertebrae ***12*** thus permit a range of movement of the vertebrae ***12*** relative to each other. **FIG. 4** illustrates a posterolateral oblique view of a vertebrae ***12*.**

The spinal cord ***40*** is a long, thin, tubular bundle of nerves ***42*** that is an extension of the central nervous system from the brain. The spinal cord ***40*** is positioned in the vertebral foramen ***19*** and protected by the bony vertebral column that forms the spinal column ***10*.** The main function of the spinal cord ***40*** is transmission of neural inputs between the periphery and the brain. Three meninges cover the spinal cord: the outer dura mater, the arachnoid mater and the innermost pia mater. Cerebrospinal fluid is found in the subarachnoid space and the spinal cord is stabilized within the dura mater by the connecting denticulate ligaments which extend from the enveloping pia mater between the dorsal and ventral roots. The lamina provides protection for the dural sac ***48*** and a foundation for the spinous processes. An epidural space ***44*** is provided between the spinal cord ***40*** and the vertebral arch ***18*** defining the vertebral foramen ***19*.** A portion of the vertebral foramen ***19*** is also occupied by the ligamentum flavum ***46*.** The ligamentum flavum ***46*** connects the lamina of adjacent vertebra ***12*.** As discussed above, however, the ligamentum flavum ***46*** can become thickened, thereby reducing the cross-sectional volume in the vertebral foramen ***19*** available to house the spinal cord ***40*.** As a result pressure is applied to the spinal cord ***40*** resulting in back pain, numbness of the legs, etc.

Thus, overall the spine ***10*** comprises a series of functional spinal units that are a motion segment surrounding the spinal cord ***40*** and which consist of two adjacent vertebral bodies ***12*,** the intervertebral disc ***34*,** associated ligaments, and facet joints ***32*.** *See* Posner, I, et al. "A biomechanical analysis of the clinical stability of the lumbar and lumbosacral spine." Spine 7:374-389 (1982).

Embodiments of the devices of the present invention include modular designs that are either or both configurable and adaptable. Additionally, the various embodiments disclosed herein may also be formed into a "kit" or system. As will be appreciated by those of skill in the art, as imaging technology improves, and mechanisms for interpreting the images (*e.g*., software tools) improve, patient specific adaptations of the tools and devices employing these concepts may be configured or manufactured prior to the surgery. Thus, it is within the scope of the invention to provide for patient specific tools and devices with integrally formed components that are pre-configured.

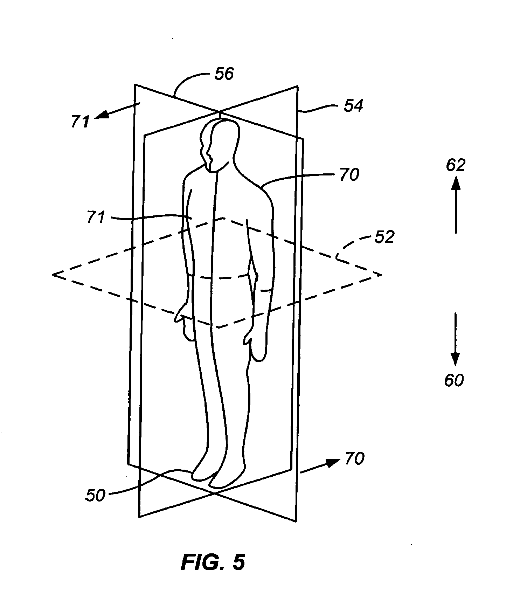

In order to understand the operational aspects of the invention, it is helpful to understand the anatomical references of the body ***50*** with respect to which the position and operation of the devices, and components thereof, are described. There are three anatomical planes generally used in anatomy to describe the human body ***50*** and structure within the human body: the axial plane ***52*,** the sagittal plane ***54*** and the coronal plane ***56*** (see **FIG. 5**). Additionally, devices and the operation of devices are better understood with respect to the caudad **60** direction and/or the cephalad direction ***62*.** Access to the body can be dorsally ***70*** (or posteriorly) such that the placement, operation or movement of the devices and tools is toward the back or rear of the body. Alternatively, devices and tools can be ventrally ***71*** (or anteriorly) such that the placement, operation or movement of the devices and tools is toward the front of the body. Various embodiments of the tools and systems of the present invention may be configurable and variable with respect to a single anatomical plane or with respect to two or more anatomical planes. For example, a tool or component thereof may be described as lying within and having adaptability in relation to a single plane. Similarly, the various components can incorporate differing sizes and/or shapes in order to accommodate differing patient sizes.

The vertebral column (spine, spinal column, backbone) forms the main part of the axial skeleton, provides a strong yet flexible support for the head and body, and protects the spinal cord disposed in the vertebral canal, which is formed within the vertebral column. The vertebral column comprises a stack of vertebrae, such as the two shown in **FIG. 3****,** with an intervertebral disc between adjacent vertebrae. The vertebrae are stabilized by muscles and ligaments that hold the vertebrae in place and limit the movements of the vertebrae.

Referring back to **FIGS. 2A** and **3****,** each vertebra ***12*** includes a vertebral body ***14*** that supports a vertebral arch ***18*.** A medial or saggital plane (**54** in **FIG. 5**) generally divides vertebra ***12*** into two substantially equal lateral sides. The vertebral body ***14*** has the general shape of a short cylinder and is anterior to the vertebral arch ***18*.** The vertebral arch ***18*** together with vertebral body ***14*** encloses a space termed the vertebral foramen ***19*.** The succession of vertebral foramen ***19*** in adjacent vertebrae ***12*** along the vertebral column define the vertebral canal (spinal canal), which contains the spinal cord ***40*.**

Vertebral arch ***18*** is formed by two pedicles ***16, 16'*** which project posteriorly to meet two laminae ***20*.** The two laminae ***20*** meet dorsal-medially to form the spinous process ***22*.** At the junction of pedicles ***16, 16'*** and laminae ***20*,** six processes arise. Two transverse processes ***24, 24'*** project dorsal and lateral, two superior articular processes ***26, 26'*** project generally superiorly and are positioned superior to two inferior articular processes ***28, 28'*** that generally project inferiorly.

The vertebral foramen ***19*** is generally an oval shaped space that contains and protects the spinal cord ***40*.** Spinal cord ***40*** comprises a plurality of nerves ***42*** surrounded by cerebrospinal fluid (CSF) and an outermost sheath/membrane called the dural sac ***48*.** The CSF filled dural sac ***48*** containing nerves ***42*** is relatively compressible. Posterior to the spinal cord ***40*** within vertebral foramen ***19*** is the ligamentum flavum ***46*.** Laminae ***20*** of adjacent vertebral arches ***18*** in the vertebral column are joined by the relatively broad, elastic ligamentum flavum ***46*.**

The vertebral foramen ***19*** contains a portion of the ligamentum flavum ***46*,** the spinal cord ***40*,** and an epidural space ***44*** between the ligamentum flavum ***46*** and the spinal cord ***40*.** The spinal cord ***40*** comprises a plurality of nerves ***42*** surrounded by cerebrospinal fluid (CSF) contained within dural sac ***48*.** Nerves ***42*** normally comprise only a small proportion of the dural sac ***48*** volume. Thus, the CSF filled dural sac ***48*** is somewhat locally compressible, as localized pressure causes the CSF to flow to adjacent portions of the dural sac. Epidural space ***44*** is typically filled with blood vessels and fat. The posterior border of the normal epidural space ***44*** generally defined by the ligamentum flavum ***46*,** which is shown in its normal, non-thickened state in **FIG. 2B****.**

**FIG. 2B** illustrates a case of spinal stenosis resulting from a thickened ligamentum flavum ***46*.** Since vertebral foramen ***19*** is defined and surrounded by relatively rigid bone, its volume is essentially constant. Thus, thickening of the ligamentum flavum ***46*** within the vertebral foramen ***19*** can eventually result in compression of the spinal cord ***40*.** In particular, the thickened ligamentum flavum ***46*** may exert a compressive force on the posterior surface of dural sac ***48*.** In addition, thickening of the ligamentum flavum ***46*** may compress the blood vessels and fat occupying the epidural space ***44*.**

Compression of the spinal cord ***40*,** particularly in the lumbar region, may result in low back pain as well as pain or abnormal sensations in the legs. Further, compression of the blood vessels in the epidural space ***44*** that houses the nerves of the cauda equina may result in ischemic pain termed spinal claudication.

In order to relieve the symptoms associated with a thickened or enlarged ligamentum flavum ***46*,** methods, techniques, and devices described herein may be employed to reduce the compressive forces exerted by the thickened ligamentum flavum on the spinal cord ***40*** and the blood vessels in epidural space ***44*** (*e.g.,* decompress spinal cord ***40*** and blood vessels in epidural space ***44***). Compressive forces exerted by the thickened/enlarged ligamentum flavum ***46*** may be reduced by a ligament decompression procedures described herein. The ligament decompression procedure is generally minimally invasive which provides benefits that will be appreciated by those skilled in the art. The ligament decompression procedure can reduce the size of the ligamentum flavum ***46*** by excising portions of the ligamentum flavum ***46*.** In some embodiments, the ligament decompression procedure may be performed percutaneously. In some embodiments of the ligament decompression procedure, the ligamentum flavum ***46*** is reduced using an ipsilateral approach of the ligament decompression procedure. Using this approach, the ligamentum flavum ***46*** can be accessed from the ipsilateral side, or the same side, of the vertebral arch ***18*.** The ligamentum flavum ***46*** can then be cut and removed ipsilaterally by a percutaneous cranial-caudal approach.

### II. TISSUE SEPARATION DEVICES

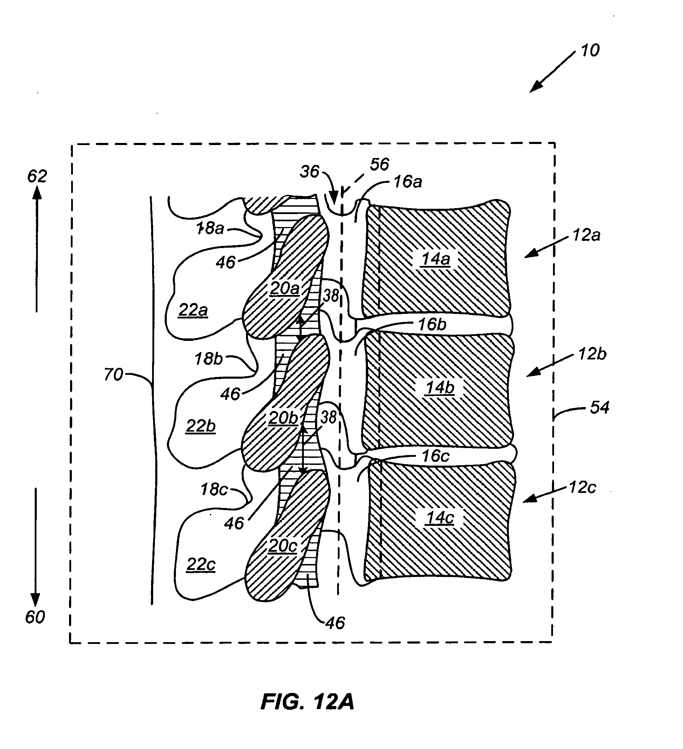

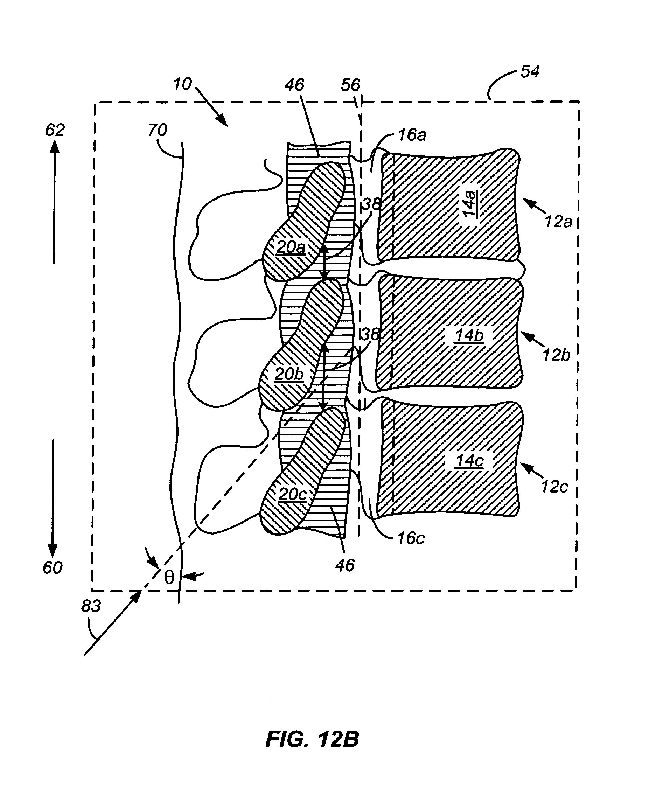

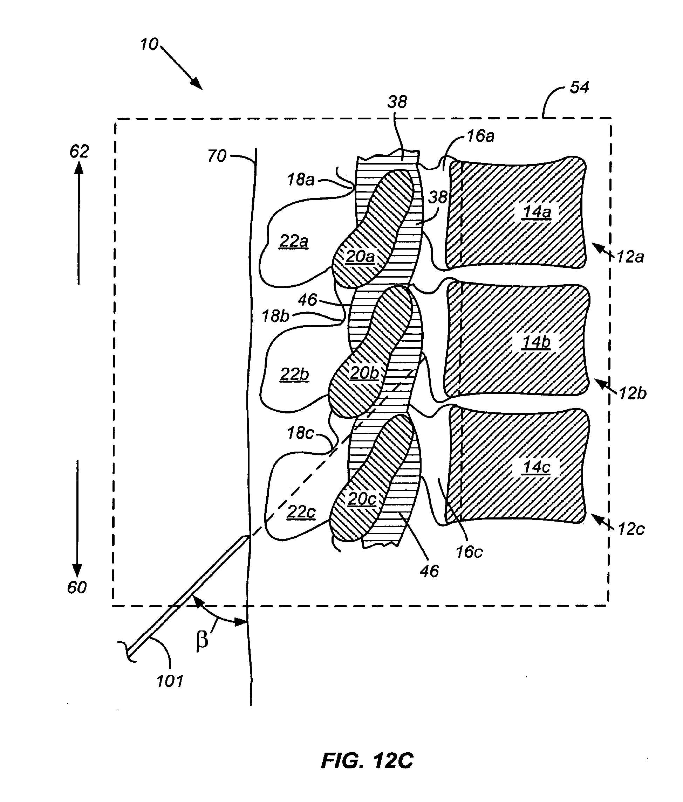

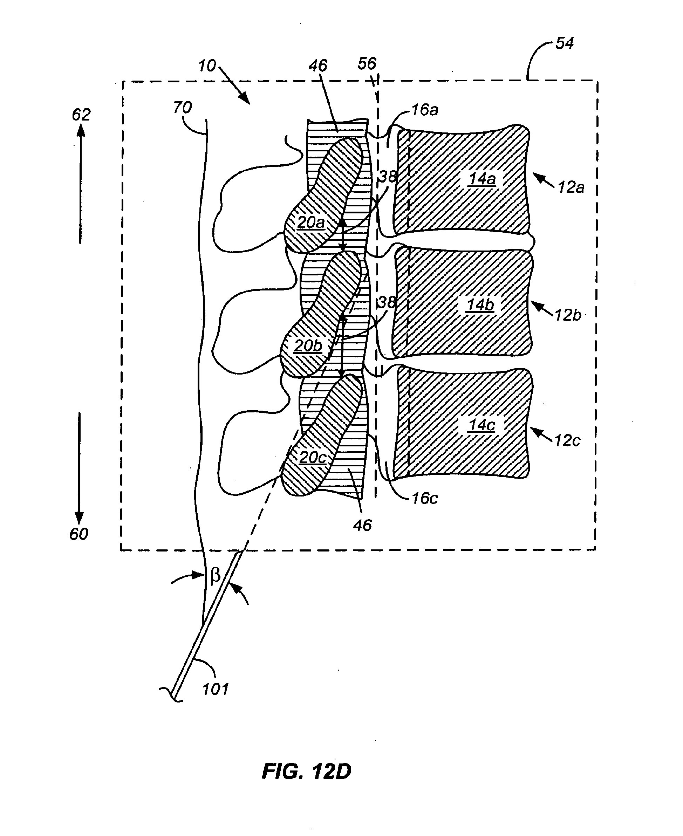

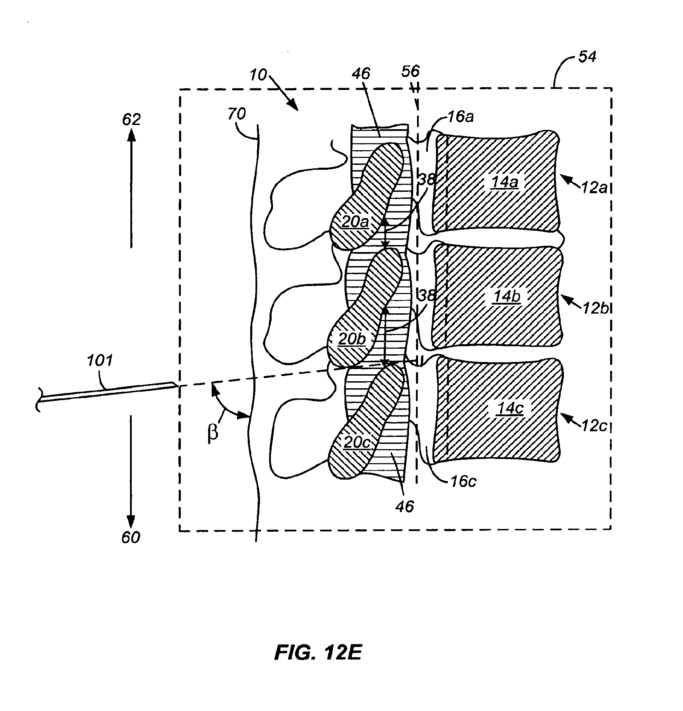

Embodiments of tissue separation tools, devices, and methods disclosed herein may take several forms and may be used according to an ipsilateral approach for minimally invasive ligament decompression procedure method described below, or used according to alternative minimally invasive ligament decompression procedures (*e.g*., minimally invasive ligament decompression procedure illustrated in **FIG. 12**). One such alternative minimally invasive ligament decompression procedure is disclosed in US 2006/0036272 which published on February 16, 2006. In the descriptions of the tissue separation devices below, the distal portions of the devices are described in detail. As will be appreciated by those skilled in the art, distal refers to positions that are relatively closer to the region of interest (*e.g*., the thickened portion of the ligamentum flavum ***46*** to be decompressed) and farthest from the point of attachment or use. In contrast, proximal refers to positions that are relatively close to point of attachment or use and furthest from the region of interest (*e.g*., the handle).

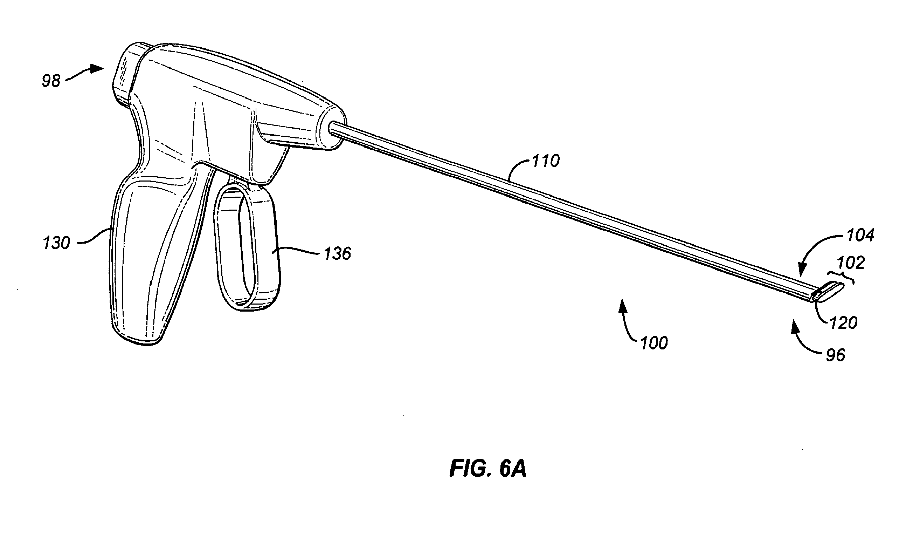

Referring now to **FIG. 6A****,** an embodiment of a device ***100*** for separating targeted tissue comprises an elongate hollow body ***110*.** Elongate inner body ***120*** is disclosed coaxially within hollow body ***110*** of device ***100*** to form tissue separation member ***102*.** A handle ***130*** is provided which is adaptable and configurable to a user to control the operation of the distal ***96*** end of the device ***100*** from its proximal ***98*** end and an actuator ***136*,** or trigger, can be provided to actuate operation of the device.

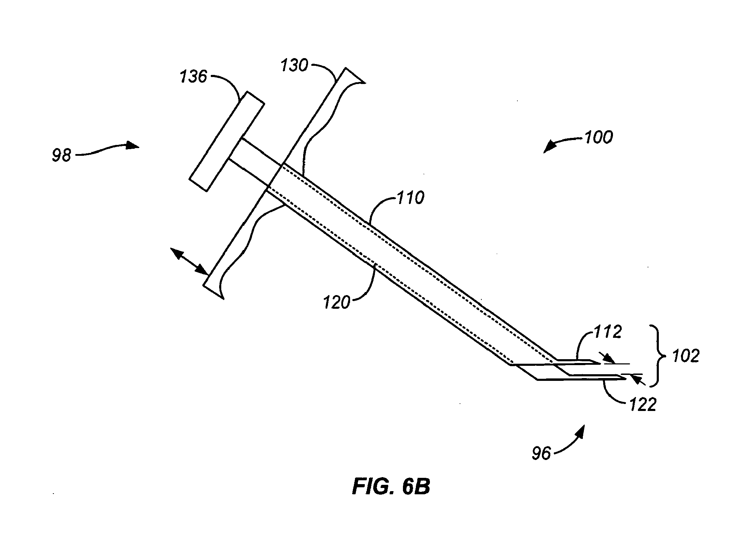

Referring now to **FIG. 6B**, elongate inner body ***120*** is disposed coaxially within the hollow body ***110*** and may be advanced or withdrawn using a variety of manual, automatic and semi-automatic mechanisms. In one example, inner member ***120*** may be coupled to a trigger or actuator ***136*,** such as an outer handle, which may be proximal to handle ***130*.** Thus, in one embodiment, handle ***130*** may be moved while outer handle ***136*** remains stationary. Alternatively, handle ***130*** may stay stationary while the outer handle ***136*** moves.

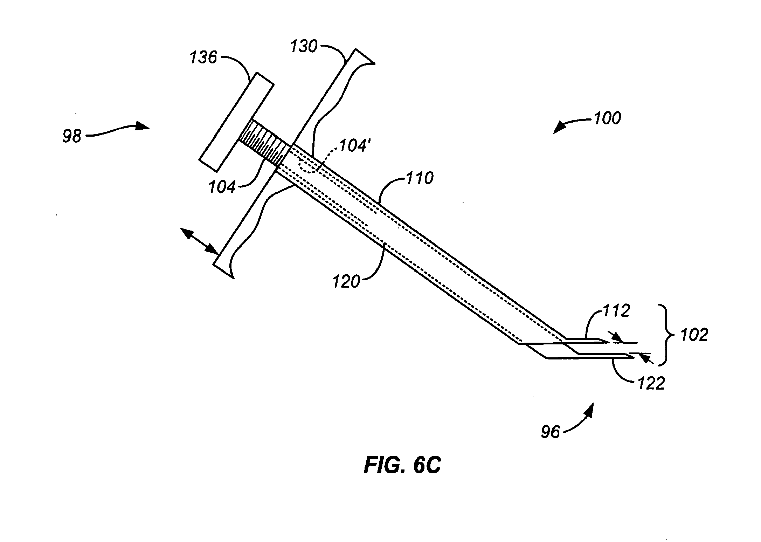

As shown in **FIG. 6C****,** the proximal end of the device ***100*** may be adapted to incorporate a threaded mechanism on the exterior surface ***104*** of at least a proximal portion of the elongate inner body ***120*** and a corresponding threaded mechanisms on the interior surface ***104'*** of at least a portion of a proximal portion of the elongate outer body ***110*.** The complementary threaded mechanisms can be calibrated to more precisely move at least one of inner member ***120*** and distal separation member ***122*** in a proximal or distal direction to facilitate separation of the components of the tissue separation member ***102*.**

It also contemplated that a ratcheting mechanism may be used to move lower or distal separation member or spreader ***122*** and/or upper or proximal separation member or spreader ***112*** incrementally apart or together.

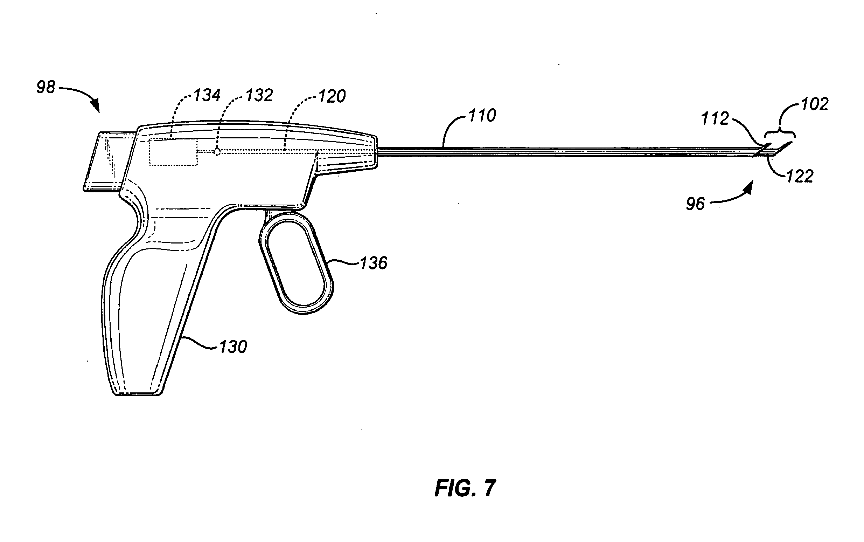

As shown in **FIG. 7****,** a drive member ***132*** can be provided that is coupled to a motor ***134*** that automatically or semi-automatically controls member ***132*,** thereby actuating one or both of the proximal separation member ***112*** and the distal separation member ***122*** of the tissue separation member ***102*.** Drive member ***132*** may be mechanically, electrically, or electromechanically coupled to tissue separation member ***102*** by any suitable mechanism including, for example, gears, frictional engagement, belts, or combinations thereof to transfer for example, a rotational torque provided by motor ***134*** to tissue separation member ***102*.** In this embodiment, motor ***134*** is enclosed in a handle ***130*** that is coupled to elongate hollow body ***110*.** Hollow body ***110*** may be releasably coupled to handle ***130*,** thereby permitting periodic access to elongate hollow body ***110*.**

In general, motor ***134*** may comprise any suitable device adaptable or configurable to drive the rotation of a drive member ***132*** and tissue separation member ***102*** including, without limitation, an electric motor, a hydraulic motor, a pneumatic motor, and the like. Motor ***134*** preferably comprises an electrical motor. In such embodiments, motor ***134*** may be powered by a rechargeable battery (*e.g*., lithium ion batteries, nickel cadmium batteries, etc.) or with electricity provided from a conventional outlet. In this embodiment, motor ***134*** is switched on and off via a trigger ***136*** operable by a finger of the individual using device ***100*.** In other embodiments, motor ***134*** may be switched on and off with a switch provided on the handle (*e.g*., handle ***130***).

In this embodiment, hollow body ***110*** is an elongate cylindrical tubular having a circular cross-section. Suitable cross-sections range, for example, from 0.1 inch to 0.5 inches. However, in general, hollow body ***110*** may have any suitable cross-sectional shape including, without limitation, hexagonal, rectangular, etc. Hollow body ***110*** may be rigid, flexible or variably rigid and flexible along its length, or adaptable and configurable to have a first configuration, *e.g*., rigid, and then a second configuration, *e.g*., flexible, as needed. Examples of suitable hollow bodies include without limitation, cannulas, hypotubes, catheters, and the like. Furthermore, hollow body ***110*** may optionally be sized to be disposed coaxially within a guiding catheter.

Although hollow body ***110*** is depicted to be a straight, elongate body, it is contemplated that hollow body ***110*** may be articulated as desired to separate a targeted tissue requiring a tortuous access path. That is, hollow body ***110*** may comprise suitable angles to permit advancement through a nonlinear path to the targeted tissue. Alternatively, hollow body ***110*** may have a smoothly curved configuration analogous to the path to be taken to access the targeted tissue.

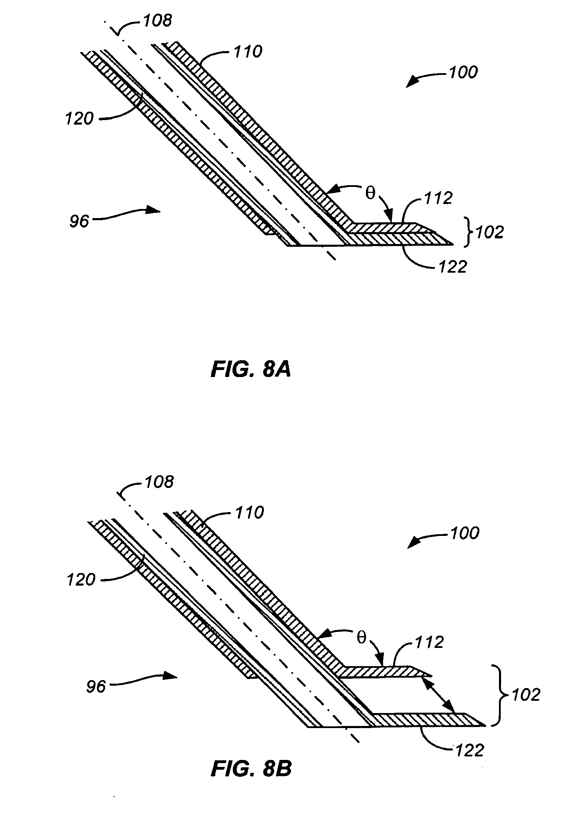

Referring now to **FIGS. 8A-B**, an embodiment of a device ***100*** for separating a tissue, such as a ligament, from another anatomical structure (*e.g*., bone, a different ligament, fat, etc.) is shown. Device ***100*** comprises an elongate hollow body ***110*** having a central longitudinal axis ***108*** and an elongate inner body ***120*** coaxially disposed within outer hollow body ***110*.** Generally, the elongate inner body has a length that is greater than its width or diameter. The distal end ***96*** of outer hollow body ***110*** includes an opening or aperture through which inner body ***120*** extends. The elongate inner body ***120*** can be hollow or solid.

Outer elongate body ***110*** further comprises a proximal separation member ***112*** that extends laterally from the outer surface of outer body ***110*** at distal end ***96*.** In this embodiment, separation member ***112*** is substantially planar, however, in other embodiments, it may be non-planar (*e.g*., curved), or any other configuration that facilitates separation of a target tissue. Separation member ***112*** is oriented at an angle θ measured relative to the longitudinal axis ***108*** of elongate body ***110*.**

Elongate inner body ***120*** further comprises a distal or lower separation member ***122*** that extends laterally from the outer surface of body ***120*** also at its distal end ***96*.** In this embodiment, separation member ***122*** is substantially planar, and is disposed substantially parallel to upper separation member ***112*.** However, in other embodiments, separation member ***122*** may be non-planar (*e.g*., curved) and/or non-parallel with upper separation member ***112*.** Separation member ***122*** is also oriented at angle θ measured relative to central longitudinal axis ***108*.**

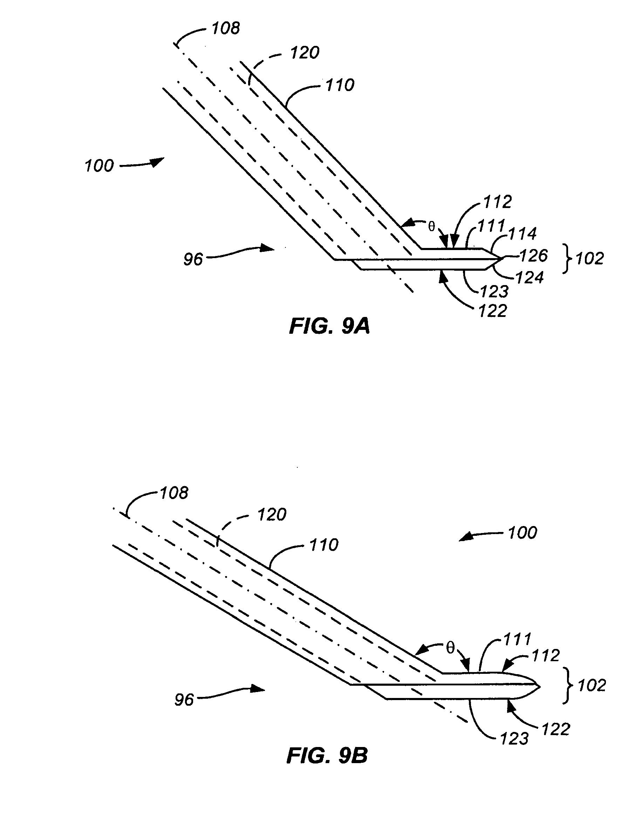

Referring still to **FIGS. 8A** and **8B****,** inner body ***120*** is slidingly disposed within outer hollow body ***110*** such that bodies ***120, 110*** may move axially relative to each other. The outer radius of inner body ***120*** is preferably substantially the same or slightly less than the inner radius of outer body ***110*,** such that inner surface of outer body ***110*** slidingly engages the outer surface of inner body ***120*.** In such embodiments, the engagement of inner surface of outer body ***110*** with the outer surface of inner body ***120*** restricts relative radial movement therebetween. As a result of the relative axial movement between bodies ***110, 120,*** lower separation member ***122*** is permitted to move axially relative to upper separation member ***112*.** More specifically, in the closed position of **FIG. 8A****,** separation members ***112, 122*** are generally disposed adjacent each other. However, in the opened position of **FIG. 8B****,** separation members ***112, 122*** are axially spaced apart. With members ***112, 122*** wedged between a tissue and another anatomical structure in the closed position **(****FIG. 9****),** and then moved axially apart by moving inner body ***120*** axially relative to outer body ***110*,** the tissue may be spaced apart from the other anatomical structure. The movement members ***112, 122*** relative to each other may be performed manually or by an actuator (not shown). Moreover the devices can be configured such that the proximal separation member ***112*** moves while the distal separation member ***122*** remains stationary, the proximal separation member ***112*** remains stationary while the distal separation member ***122*** moves, or both the proximal and distal separation members are capable of concurrent or serial movement.

In the embodiment shown in **FIG. 9A** and **9B****,** outer hollow body ***110*** is an elongate cylindrical tubular having a circular cross-section. However, in general, outer hollow body ***110*** may comprise any suitable cross-sectional shape including, without limitation, hexagonal, rectangular, etc. Examples of suitable hollow bodies include without limitation, cannulas, hypotubes, catheters, and the like. Further, outer hollow body ***110*** may be rigid or flexible as desired, or may have rigid sections and flexible sections along its length. In some embodiments, device ***100*** may be advanced to the region of interest via a guiding catheter.

Inner body ***120*** may either be hollow or solid. As with outer hollow body ***110*,** inner body ***120*** may have any suitable cross-sectional shape including, without limitation, hexagonal, rectangular, etc. Inner body ***120*** may be made of the same material or a different material as outer hollow body ***110*.** Preferably, inner body ***120*** is made of a flexible material.

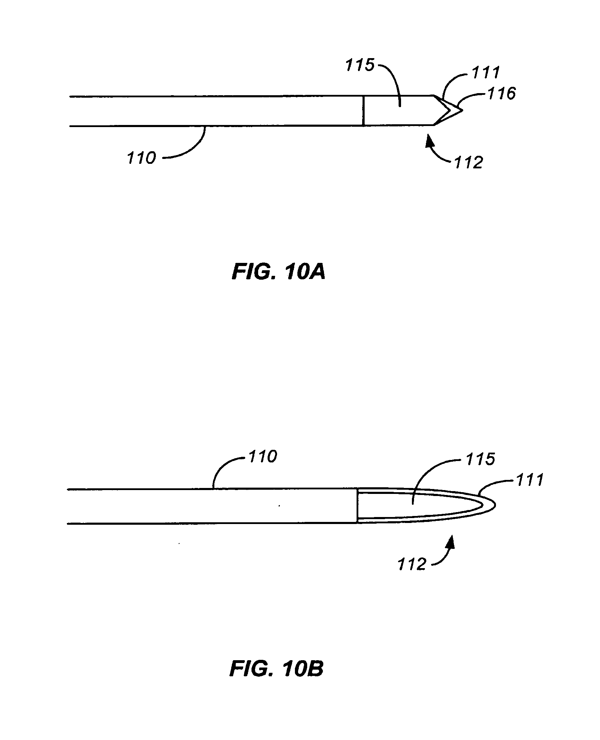

As discussed above, outer hollow body ***110*** and inner body ***120*** have an upper separation member ***112*** and a lower separation member ***122*,** respectively for facilitating the separation of a tissue from another anatomical structure (*e.g*., bone). When viewed in profile, upper and lower separation members ***112, 122,*** may be configured in any number of geometrical configurations. As shown in **FIG. 9A****,** outer face ***111*** of upper members ***110,*** and outer face ***123*** of inner member ***120*** are flat or planar and have beveled outer edges ***114, 124*** to facilitate insertion of the device ***100*** between tissue and structure to be spaced from the ligament. Beveled edges ***114, 124*** may either be alternatively angled to form a point as show in **FIG. 9A****,** or may be angled to form a slope as seen in **FIG. 9B****.** Alternatively, upper and lower members ***112, 122*** may have curved convex surfaces as shown in **FIGS. 9B** and **10B****.** Separation members ***112, 122*** can be configured to have a concave configuration for assisting in separation of ligaments and/or bone. Moreover, separation members ***112, 122*** can be symmetric in side view as seen in **FIG. 9A-B**. However, it is contemplated that some embodiments of device ***100*** may have asymmetric separation member ***112, 122.*** For example, upper separation member ***112*** may have a flat outer surface ***111*** while outer face ***123*** of lower separation member may have a convex, curved surface, and vice versa.

As previously described, both upper (proximal) and lower (distal) separation members ***112, 122*** are disposed at an angle θ relative to central longitudinal axis ***108.*** Angle θ is preferably an obtuse angle (i.e. greater than about 90 degrees). More specifically, angle θ preferably ranges from about 90 degrees to about 175 degrees, and more preferably ranges from about 110 degrees to about 150 degrees. Upper and lower separation members ***112, 122*** may be integral with bodies ***110, 120,*** respectively, or coupled to bodies ***110, 120,*** respectively. In some embodiments, one or both of members ***112, 122*** may be coupled to bodies ***110, 120,*** respectively, such that angle θ may be adjusted as needed. In the open configuration shown in **FIG. 8B****,** upper and lower separation members ***112, 122*** may be adjusted to be open at any suitable axial distance apart. The distance between upper and lower separation members ***112, 122*** in the open configuration preferably ranges from about 3 mm to about 16.

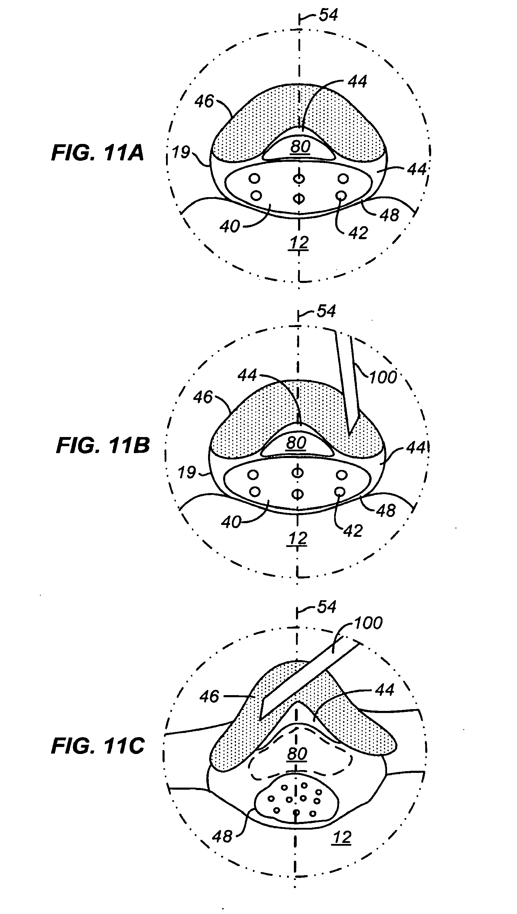

**FIGS. 10A-B** show the top view of different embodiments of the device ***100.*** Outer edge ***111*** of upper separation member ***112*** and outer edge ***121*** (not shown) of lower separation member ***122*** (not shown) may be angled or tapered for better insertion of device between tissue and bone. In other embodiments, outer edge ***111*** and outer edge ***121*** (not shown) of upper separation member ***112*** and lower separation member ***122*** (not shown), respectively, may have a curved aspect as shown in **FIG. 11B****.** Outer face ***115*** and outer face ***125*** (not shown) of upper separation member ***112*** and lower separation member ***122*** (not shown), respectively, may comprise a texture such as a knurling/diamond texture or a nodular texture to provide enhanced grip of tissue and/or bone. As will be appreciated by those skilled in the art, the outer edge ***121*** and outer face ***125*** are positioned beneath the outer edge ***111*** and outer face ***115*** in these figures.

Referring back to **FIG. 6B****,** proximal end of outer hollow body may be coupled to inner handle. Inner member ***120*** may be advanced using a variety of mechanism. More particularly, inner member ***120*** may be coupled to an outer handle ***136,*** which may be proximal to inner handle ***130.*** Thus, inner handle ***130*** may be moved while outer handle ***136*** remains stationary. Alternatively, proximal end of device ***100*** may incorporate a threaded mechanism as described above to more precisely move inner member ***120*** and lower separation member ***122*** in a distal direction. It also contemplated that a ratcheting mechanism may be used to move lower separation member ***122*** and/or upper separation member ***112*** incrementally apart or together.

### III. METHODS OF TREATMENT

### A. Creation of a Safety Zone

As shown in **FIG. 2B****,** the ligamentum flavum ***46*** is posteriorly apposed to the spinal cord ***40.*** The ligamentum flavum ***46*** can become enlarged. The placement of tools within the ligamentum flavum ***46*** to separate portions of the ligamentum flavum ***46*** creates a risk of inadvertent damage to the spinal cord ***40,*** dural sac ***48,*** and/or nerves ***42.*** Thus, in some embodiments of the procedures described herein, prior to insertion of tissue separation devices into the ligamentum flavum ***46,*** a gap, pocket, or space is advantageously created between the ligamentum flavum ***46*** and the spinal cord ***40*** to provide a safety zone ***80*** (illustrated in **FIG. 11**) between the ligamentum flavum ***46*** and the spinal cord.

The vertebral foramen ***19*** includes the epidural space ***44*** and spinal cord ***40*** containing nerves ***42*** and CSF within the dural sac ***48.*** Further, a thickened/enlarged ligamentum flavum ***46*** can, as will be appreciated by those skilled in the art, extend into the vertebral foramen ***19.*** To reduce the risk of damage to the dural sac ***48*** and the spinal cord ***40,*** a safety zone is created between the ligamentum flavum ***46*** and the dural sac ***48*** according to the methods disclosed herein.

As previously described, the spinal cord ***40*** comprises nerves ***42*** surrounded by CSF, and is contained within the dural sac ***48.*** Since more than 90% of the volume of the dural sac ***48*** in the lumbar region is filled by CSF, the dural sac ***48*** is highly compressible. Thus, even when stenosis is causing compression of the spinal cord ***40,*** in most cases it is possible to temporarily compress spinal cord ***40*** even further. The dural sac ***48*** can be further compressed in the region of interest by introducing a media into the epidural space ***44*** to create a safety zone. For example, the media can be a fluid, a gel, or any other suitable media for compressing the spinal cord. The media can be introduced into the epidural space ***44*** with an insertion member, such as a needle, catheter, cannula, or any other suitable insertion device. The media located in the safety zone gently applies an additional compressive force to the outer surface of the dural sac ***48*** so that at least a portion of the CSF within dural sac ***48*** is forced out of the dural sac ***48*** in the region of interest, resulting in a safety zone between the dural sac ***48*** and the ligamentum flavum ***46.***

According to some embodiments, the dural sac ***48*** can be compressed by introducing, for example, a contrast medium into the region of interest in the epidural space ***44.*** The introduction of the contrast medium can provide contrast guided dural protection. Additionally, the contrast medium can be used to create a safety zone or to aid in the visualization of the surgical area. In some embodiments, the contrast medium can be used to both create the safety zone and to aid in imaging the region of interest. The contrast medium can be a standard radio-opaque non-ionic myelographic contrast medium or any other suitable imagable or non-imagable contrast medium. The contrast medium can be introduced into the epidural space by injection of the contrast medium. In some embodiment, the injection is a percutaneous injection. A sufficient amount of contrast media can be injected into the region of interest in the epidural space ***44*** to displace the CSF out of the region of interest and to compress the dural sac ***48.*** The material can compress the dural sac ***48*** entirely. Alternatively, the material can compress the dural sac ***48*** partially. The dural sac ***48*** can be compressed to any desired degree. Once introduced into the region of interest, the introduced media can be entirely contained within the confines of the epidural space ***44.*** At the same time, the media extends to the margins of the dural sac ***48.*** Alternatively, the introduced media can be partially contained within the confines of the epidural space ***44.*** The epidural space ***44*** is substantially watertight and the fatty tissues and vascularization in epidural space ***44,*** combined with the viscous properties of the contrast medium, serve to substantially maintain the injected medium in the desired region of interest.

Once a safety zone ***80*** has been created, a tissue separation tool or device ***100,*** such as those described above, may be inserted into the ligamentum flavum ***46.*** Device ***100*** may comprise any suitable device, tool or instrument for relieving stenosis caused by the thickened/enlarged ligamentum flavum ***46*** including without limitation, embodiments of tissue separation devices and tissue retraction devices described herein. In some embodiments, device ***100*** is inserted and positioned in the ligamentum flavum ***46*** on the same side (ipsilateral) of the sagittal plane ***54*** as device ***100*** percutaneously accesses the body, such that device ***100*** does not cross the sagittal plane ***54*** as shown in **FIG. 11B****.** Alternatively, device ***100*** can be positioned in the ligamentum flavum ***46*** on the opposite side of sagittal plane ***54*** as device ***100*** percutaneously accesses the body, such that device ***100*** crosses the sagittal plane ***54*** as shown in **FIG. 11C****.** In some embodiments, the tissue separation device ***100*** can be guided by and advanced through a cannula toward the ligamentum flavum ***46.*** In some embodiments, the device ***100*** can be advanced toward the ligamentum flavum ***46*** without the use of a cannula.

While it is preferred that the tip of device ***100*** remain within the ligamentum flavum ***46*** as shown, the presence of the safety zone reduces the likelihood that the dural sac ***48*** will be damaged, even if the tip of device ***100*** breaks through the anterior surface of the ligamentum flavum ***46.***

Because the present techniques are preferably performed percutaneously, certain aspects of the present disclosure may be facilitated by imaging. Imaging windows (*e.g*., a fluoroscopic window of access) can be employed to aid in performance of all or part of the procedures described herein. For instance, an imaging window can be employed to aid in insertion of device ***100*** into the ligamentum flavum ***46.***

The spine can be imaged using any suitable technology including, without limitation, 2D fluoroscopy, 3D fluoroscopy, CT, MRI, and ultrasound. The spine can also be directly visualized using fiber optic or microsurgical techniques. Stereotactic or computerized image fusion techniques are also suitable for imaging the spine. Fluoroscopy is currently particularly well-suited to the techniques disclosed herein. Fluoroscopic equipment is safe and easy to use, readily available in most medical facilities, and relatively inexpensive. In a typical procedure, using direct biplane fluoroscopic guidance and local anesthesia, the epidural space ***44*** is accessed for injection of contrast media adjacent to the surgical site. In some instances, the procedure can be performed without imaging the spine in advance.

If the injected medium is radio-opaque, as are for example myelographic contrast media, the margins of the expanded epidural space ***44*** will be readily visible using fluoroscopy or CT imaging. Thus, the safety zone created by the present contrast-guided dural compression techniques can reduce the risk of damage to the dural sac ***48*** and the spinal cord ***40*** during ligament decompression procedures to remove or displace portions of the ligamentum flavum ***46*** and/or laminae ***20*** in order to treat spinal stenosis.

### B. Use of Injectable Medium

In one aspect of the invention, the medium introduced into the epidural space ***44*** can be a gel, including, but not limited, to a re-sorbable water-soluble gel. A gel can be used to localize the safety zone ***80*** at the site of surgery and to reduce leakage of the contrast medium from the protective layer from the vertebral/spinal canal ***36.*** In some embodiments, the contrast medium can be an injectable gel. The gel can be more viscous than conventional contrast media. The viscosity of the gel enables the gel to be localized at the desired region of interest. This is in contrast to standard liquid contrast media that are used in epidurography, which have more of a tendency to spread out from the region where injected. The use of a gel can result in more uniform compression of the dural sac ***48*** and less leakage of contrast medium out of the vertebral/spinal canal. In addition, contrast gels can be more slowly re-absorbed allowing for better visualization of the region of interest during the entire course of the surgical procedure. In some embodiments, an amount of gel is introduced as is necessary to compress the dural sac a desired amount. In some embodiments, an expandable gel is introduced. The gel can expand to fill the epidural space and to compress the dural sac. In some embodiments, the gel is re-absorbed at a rate allowing for better visualization of the region of interest during part of the surgical procedure.

A gel can be introduced into the epidural space ***44.*** The gel can either comprise a contrast agent or a contrast agent can be introduced in the epidural space ***44*** simultaneously with the gel. An amount of contrast agent can be introduced into the epidural space followed by an amount of gel. Alternatively, an amount of gel can be introduced into the epidural space followed by an amount of contrast agent.
The contrast agent can be captured on the surface of the gel mass, so that the periphery of the gel mass is imagable.

In some embodiments, a copolymer gel can be used including, but not limited to, standard hydrophilic-lipophilic block copolymer gel, or any other suitable gel can be used. In some embodiments, the gel comprises an inert base. The gel material can be a temperature dependent gel material. In some embodiments, the gel can be a liquid at ambient temperatures and can be injected into the epidural space through a small bore, such as a 27 gauge needle. When warmed to body temperature, the gel can thicken thereby becoming more viscous. The viscosity of the gel can also be adjusted through the specifics of the preparation of the gel. In some embodiments, the injected gel attains a viscosity that is two, three, six or even ten times that of the fluids that are typically used for epidurograms. The gel or other fluid can be sufficiently viscid or viscous at body temperature to compress and protect dural sac ***48*** in the manner described above. In some embodiments, the gel can remain in the region of interest for at least about thirty (30) minutes after being injected into the epidural space.

In certain embodiments, the injected medium undergoes a reversible change in viscosity when warmed to body temperature so that it can be injected as a low-viscosity fluid, thicken upon injection into the patient, and be returned to its low-viscosity state by cooling. In some embodiments, the injected medium is introduced to the epidural space as desired and the gel thickens upon being warmed by the body temperature. In some embodiments, the gel can be removed by contacting the gel with a heat removal device, such as an aspirator that has been provided with a cooling tip, needle, catheter, or other suitable cooling device. As a result of localized cooling, the gel can revert to its initial non viscous liquid state and can be easily suctioned up by the aspirator, or other suitable suction device.

An example of a suitable contrast medium having the desired properties is OMNIPAQUE® 240 (iohexol) available from Nycomed, New York, which is a commercially available non-ionic iodinated myelographic contrast medium. Other suitable media will be known to those skilled in the art. Because of the proximity to the spinal cord ***40*** and spinal nerves ***42,*** it is preferred not to use ionic media in the media. In some embodiments, the compositions are reabsorbed relatively rapidly after the procedure and any residual compression on the dural sac ***48*** after the ligament decompression procedure dissipates relatively quickly. For example, in some embodiments, the gel can have sufficient viscosity to compress the dural sac ***48*** for thirty (30) minutes, and sufficient degradability to be substantially reabsorbed within approximately two hours.

The introduced contrast medium can further comprise one or more bioactive agents. For example, medications such as those used in epidural steroid injections (*e.g.*, Depo Medrol® (methylprednisolone acetate), Celestone® Soluspan® (betamethasone sodium phosphate and betamethasone acetate) can be added to the epidural gel to speed healing and reduce inflammation, scarring, and adhesions. The gel can release the steroid medication slowly and prolong the anti-inflammatory effect, which can be extremely advantageous. Local anesthetic agents can also be added to the gel. This prolongs the duration of action of local anesthetic agents in the epidural space to prolong pain relief during epidural anesthesia. In this embodiment the gel can be formulated to slow the re-absorption of the gel.

The gels can also be used for epidural steroid injection and perineural blocks for management of acute and chronic spinal pain. Thrombin or other haemostatic agents can be added if desired, so as to reduce the risk of bleeding.

In some embodiments, the gel can also be used as a substitute for a blood patch if a CSF leak occurs. The gel can also be used as an alternative method to treat lumbar puncture complications such as post-lumbar puncture CSF leak or other causes of intracranial hypotension. Similarly, the gel can be used to patch postoperative CSF leaks or dural tears. If the dural sac is inadvertently torn or cut, the gel can serve to immediately seal the site and prevent leakage of the cerebral spinal fluid.

### C. Imaging Techniques

As will be appreciated by those of skill in the art, imaging techniques suitable for assessing spinal stenosis and evaluating the epidural space include the use of x-rays, magnetic resonance imaging (MRI), computed tomography scanning (CT, also known as computerized axial tomography or CAT), optical coherence tomography, SPECT, PET, ultrasound imaging techniques, and optical imaging techniques. (See, also, US Patent 6,373,250 to Tsoref et al.; and Vandeberg et al. (2002) Radiology 222:430-436). Contrast or other enhancing agents can be used using any route of administration, e.g. intravenous, intra-articular, etc.

In certain embodiments, CT or MRI is used to assess tissue, bone, cartilage and any defects therein, and to provide morphologic or biochemical or biomechanical information about the target area of interest. For discussions of the basic NMR principles and techniques, see MRI Basic Principles and Applications, Second Edition, Mark A. Brown and Richard C. Semelka, Wiley-Liss, Inc. (1999). Two-dimensional, three-dimensional images, or maps, of the target area alone or in combination with a movement pattern, e.g. flexion-extension, translation and/or rotation, can be obtained. Three-dimensional images can include information on movement patterns, contact points, contact zone of two or more opposing surfaces, and movement of the contact point or zone during joint motion. Two and three-dimensional images can include information on biochemical composition of the articular cartilage. In addition, imaging techniques can be compared over time, for example to provide up-to-date information on the shape and type of repair material needed.

Any of the imaging devices described herein can also be used intra-operatively, for example using a hand-held ultrasound and/or optical probe to image the target area intra-operatively.

### D. Approaching the Stenosis Ipsilaterally for Minimally Invasive Ligament Decompression Procedures

Once the safety zone ***80*** has been created, the margins of the epidural space ***44*** are clearly demarcated by the introduced medium and can be visualized radiographically if an imageable medium has been used. As mentioned above, percutaneous procedures can be performed more safely on the ligamentum flavum ***46*** and/or surrounding tissues while reducing the potential for injuring the dural sac ***48*** and the spinal cord ***40.*** As shown in **FIGS. 11A-C**, and discussed above, the ligamentum flavum ***46*** can be accessed ipsilaterally or contralaterally.

A variety of suitable techniques and devices may be employed to reduce the size of the thickened/enlarged ligamentum flavum ***46,*** thereby decompressing the spinal cord ***40*** as well as blood vessels contained within the epidural space ***44.*** Examples of suitable decompression techniques include without limitation, removal of tissue from the ligamentum flavum ***46,*** laminectomy, laminotomy, and retraction and anchoring of the ligamentum flavum ***46.*** In some embodiments, all or a portion of the ligamentum flavum ***46*** is separated using a tissue separation device or tool (*e.g*., devices ***100*** described herein).

Accessing the ligamentum flavum ***46*** with one of the tissue separation devices ***100*** to remove portions of the ligamentum flavum ***46*** can present significant challenges. For instance, in some conventional approaches to correct stenosis caused by an enlarged ligamentum flavum ***46,*** an incision is made in the back of the patient and then the muscles and supporting structures of the vertebral column (spine) are stripped away, exposing the posterior aspect of the vertebral column. Subsequently, the thickened ligamentum flavum ***46*** is exposed by removal of a portion of the vertebral arch ***18,*** often at lamina ***20,*** which encloses the anterior portion of the spinal canal (laminectomy). The thickened ligamentum flavum ***46*** can then be excised by sharp dissection, *e.g*., with a scalpel or punching instruments. However, this approach is usually performed under general anesthesia and typically requires an extended hospital stay, lengthy recovery time and significant rehabilitation. As another example, some ligament decompression procedures access the ligamentum flavum ***46*** percutaneously by boring a hole through the vertebral arch ***18*** of the vertebra ***12,*** often through a lamina ***20.*** A cannula and/or device ***100*** may be passed through the bore and/or anchored to the bore to access ligamentum flavum ***46*** for modification and/or separation. However, while such a ligament decompression procedure is minimally invasive and reduces recovery time, such an approach requires the additional step of boring a hole in the posterior of the vertebra ***12*** of interest. Thus, in some cases it will be preferable to employ a ligament decompression procedure that percutaneously accesses the ligamentum flavum ***46*** without the need to cut or bore through the vertebrae.

**FIGS. 12A-E** are a partial cross-sectional lateral view of a segment of a spinal column ***10.*** The partial cross-sectional view is taken across a sagittal plane ***54.*** The segment of spinal column ***10*** illustrated in FIG. 12A includes three vertebrae ***12a, 12b,*** and ***12c.*** Each vertebra ***12a, 126, 12c*** includes a vertebral body ***14a, 14b, 14c,*** that supports a vertebral arch ***18a, 18b, 18c,*** respectively. Vertebral body ***14a, 14b, 14c*** is anterior to vertebral arch ***18a, 18b, 18c,*** respectively. Each vertebral arch ***18a, 18b, 18c*** together with vertebral body ***14a, 14b, 14c,*** respectively, encloses a vertebral foramen. The succession of vertebral foramen in adjacent vertebrae ***12a, 12b, 12c*** defines vertebral canal ***36*** (spinal canal) that runs along the length of vertebral column ***10*** and which is illustrated along the length of the intersection between the sagittal ***54*** and coronal ***56*** planes. Vertebral canal ***36*** contains the spinal cord (not shown in **FIG. 12**).

As previously described, each vertebral arch ***18a,18b,18c*** includes two pedicles ***16a, 16b, 16c,*** which project posteriorly to meet two lamina ***20a, 20b, 20c,*** respectively. It is to be understood that in this view, one pedicle has been removed from each vertebra ***12a, 12b, 12c*** and only the cross-section of one lamina ***20a, 20b, 20c*** is visible. The two lamina ***20a, 20b, 20c*** meet dorsal-medially to form the spinous process ***22a, 22b, 22c,*** respectively.

Lamina ***20a, 20b, 20c*** of adjacent vertebra ***12a, 12b, 12c*** are connected by the ligamentum flavum ***46*** (shown in cross-section). The relatively elastic ligamentum flavum ***46*** extends almost vertically from the superior lamina to the inferior lamina of the adjacent vertebrae. In particular, the ligamentum flavum ***46*** originates on the inferior surface of the laminae of the superior vertebrae and connects to the superior surface of the laminae of the inferior vertebrae. The ligamentum flavum ***46*** originates on the inferior surface of lamina ***20a*** of superior vertebra ***12a*** and connects to the superior surface of lamina ***20b*** of the inferior vertebra ***12b.*** Thus, the ligamentum flavum ***46*** spans an interlaminar space ***38*** (*i.e.,* space between laminae of adjacent vertebrae). The interlaminar space ***38*** is generally the space between laminae of adjacent vertebrae in the spinal column ***10.***

Still referring to **FIGS. 12B-D**, each lamina ***20a, 20b, 20c*** comprises a relatively broad flat plate of bone that extends dorsal-medially and slightly inferiorly from pedicles ***16a, 16b, 16c,*** respectively. Along the length of vertebral column ***10,*** the lamina ***20a, 20b, 20c*** overlap, with each lamina substantially parallel to and at least partially overlapping the adjacent inferior lamina. Further, the adjacent substantially parallel laminae are separated by the intervening ligamentum flavum ***46*** and the interlaminar space ***38.*** For instance, the lamina ***20a*** is substantially parallel to and partially overlaps adjacent inferior lamina ***20b*** and is separated from lamina ***20b*** by the ligamentum flavum ***46*** and the interlaminar space ***38.***

FIG. 12E illustrates vertebral column ***10*** as may be encountered during a spinal procedure or surgery. In addition, in the embodiment illustrated in **FIG. 12E**, the ligamentum flavum ***46*** is thickened/enlarged, resulting in spinal stenosis. In particular, the anterior portions of the enlarged ligamentum flavum ***46*** extend into spinal canal ***36,*** potentially exerting compressive forces on the spinal cord (not shown) that resides within spinal canal ***36.***

As previously discussed, to relieve compressive forces on the spinal cord and hence relieve the associated symptoms of spinal stenosis, portions of the ligamentum flavum ***46*** may be separated. However, to percutaneously separate portions of the ligamentum flavum ***46*** via minimally invasive techniques, the innate structure of vertebral column ***10*** and each vertebra ***12*** may present significant imaging challenges. For instance, lateral imaging windows/views of the ligamentum flavum ***46*** substantially in the coronal plane ***56*** may be obscured by the various processes of the vertebrae (*e.g*., transverse processes, superior articular processes, inferior articular processes), and the laminae of each vertebra, etc. Further, some anterior-posterior (A-P) imaging windows/views of the ligamentum flavum ***46*** substantially in the sagittal plane ***54*** may also be obscured by the laminae ***20.*** In particular, in the A-P radiographic imaging planes substantially in the sagittal plane ***54,*** the posterior edges of parallel laminae ***20*** overlap and obscure the ligamentum flavum ***46*** and the interlaminar space ***38,*** particularly the anterior portions of the ligamentum flavum ***46*** and the interlaminar space ***38*** closest to spinal canal ***36.*** However, with an imaging window/view in a plane substantially parallel to the sagittal plane ***54,*** at an angle generally in the direction of arrow ***83*** shown in FIG. 12B, and slightly lateral to the spinous process ***22,*** interlaminar space ***38*** and ligamentum flavum ***46*** may be viewed without significant obstruction from neighboring laminae ***20.*** In other words, imaging windows/views generally aligned with arrow ***83*** (**FIG. 12B**) allow for a more direct view of the interlaminar space ***38*** and the ligamentum flavum ***46*** from the posterior back surface with minimal obstruction by the vertebrae, or from the laminae.

Typically, the long axes of the substantially parallel laminae (*e.g*., laminae ***20a, 20b, 20c)*** and interlaminar spaces (*e.g*., interlaminar spaces ***38***) are generally oriented between about 60 and about ***75*** degrees relative to posterior back surface ***70.*** Thus, preferably the imaging means (*e.g*., x-ray beam, fluoroscopy tube, etc.) is positioned generally in the direction represented by arrow ***83,*** where θ is substantially between about ***60*** and about ***75*** degrees relative to the anterior back surface ***70.*** In other words, the imaging apparatus is positioned substantially parallel to the surface of the laminae. The resulting imaging window/view, termed "caudal-cranial posterior view" hereinafter, permits a clearer, more direct, less obstructed view of the interlaminar space ***38*** and the ligamentum flavum ***46*** from the general posterior back surface ***70.*** The caudal-cranial posterior view permits a relatively clear view of the interlaminar space ***38*** and the ligamentum flavum ***46*** in directions generally along the axial and coronal planes. However, the caudal-cranial posterior view by itself may not provide a clear imaging window/view of the interlaminar space ***38*** and the ligamentum flavum ***46*** in directions generally along the sagittal plane ***54.*** In other words, the caudal-cranial posterior view by itself may not provide a clear imaging window/view that can be used to accurately determine the posterior-anterior depth, measured generally along the sagittal plane, of a device across the ligamentum flavum ***46.***

In some embodiments, an additional imaging window/view, termed "caudal-cranial posterior-lateral view" hereinafter, is employed to provide a clearer, unobstructed view of interlaminar space ***38*** and ligamentum flavum ***46*** in directions generally along the axial ***52*** and coronal ***56*** planes. The caudal-cranial posterior-lateral view is generated by orienting an imaging means generally at an angle θ relative to the outer surface of the patient and also angling such imaging means laterally in an oblique orientation, revealing a partial lateral view of the interlaminar space ***38*** occupied by the ligamentum flavum ***46*** on the anterior side of the lamina and posterior to the underlying dural sac (not shown) and spinal cord (not shown).

By employing at least one of the caudal-cranial posterior views and the caudal-cranial posterior-lateral views, relatively clear imaging windows/views of the interlaminar space ***38*** and ligamentum flavum ***46*** in directions along the sagittal ***54,*** coronal ***56,*** and axial ***52*** planes may be achieved.

**FIGS. 12C-E** illustrate vertebral column ***10*** and an instrument ***100.*** Once unobstructed imaging windows/views of the interlaminar space ***38*** and the ligamentum flavum ***46*** are established in the manner described above, instrument ***100*** is employed to percutaneously access the interlaminar space ***38*** and the ligamentum flavum ***46.*** Instrument ***100*** may be any suitable device necessary to perform the ligament decompression procedures described herein including without limitation a tissue separation device, a cannula employed to guide a tissue separation device, or combinations thereof. Tissue separation tools and devices are described in more detail below.

More specifically, using images of the interlaminar space ***38*** and the ligamentum flavum ***46*** obtained from the desired direction(s), (*e.g*., caudal-cranial posterior view and the caudal-cranial posterior-lateral view), instrument ***100*** can be employed to penetrate the skin and soft tissue in the posterior back surface ***70*** of the patient. In preferred embodiments, the skin entry point for instrument ***100*** is between about 5 and about 10 cm inferior (caudal to) the posterior surface of the interlaminar space ***38*** of interest. For instance, if the portion of the ligamentum flavum ***46*** between lamina ***20a*** and lamina ***20b*** is the area of interest, then instrument ***100*** may be inserted into the patient's back about 5 to about 10 cm inferior to posterior surface ***70*** of the interlaminar space ***38.***

Referring still to **FIGS. 12C-E**, instrument ***100*** can be initially inserted into the posterior tissue and musculature of the patient generally parallel to the longitudinal axis of spinal column ***10.*** In other words, the angle β between the posterior back surface ***70*** and instrument ***100*** is between about 0 and about 10 degrees when instrument ***100*** is initially inserted. Further, instrument ***100*** is preferably inserted into the posterior tissue and musculature of the patient on the same side (ipsilateral) of the median plane as the area of interest (*e.g*., the targeted portion of ligamentum flavum ***46***), as best seen in FIG. 11B. Once instrument ***100*** is inserted into the posterior tissue and musculature of the patient, instrument ***100*** then may be oriented about 5 to about 90 degrees relative to the posterior back surface ***70*** of the patient in order to create a trajectory across the ligamentum flavum ***46*** in the area of interest (*see, e.g.,* **FIGS. 12C-E**). Furthermore, once an instrument is inserted into the patients posterior back surface ***70,*** the ends of instrument ***100*** are free to pivot about the insertion location in posterior back surface ***70*** in the general direction of the axial ***52*** and the coronal ***56*** planes, and may be advanced posteriorly or anteriorly generally in the direction of the sagittal ***54*** plane.

Once inserted into the posterior tissue and musculature of the patient, instrument can be positioned to provide a trajectory across the interlaminar space ***38*** in the area of interest, generally towards the anterior ***71*** surface of the lamina ***20*** superior to the area of interest. For example, if interlaminar space ***38*** between lamina ***20a*** and lamina ***20b*** is the area of interest, instrument ***100*** is positioned to provide a trajectory that will allow a cutting instrument to be inserted across interlaminar space ***38*** between one lamina ***20a*** and another lamina ***20b*** towards the anterior surface of lamina ***20a*** (superior on cephalad ***62*** lamina).

By switching between the caudal-cranial posterior view and the caudal-cranial posterior-lateral view, or by viewing both the caudal-cranial posterior view and the caudal-cranial posterior-lateral view at the same time, the instrument can be advanced to the ligamentum flavum ***46*** in the area of interest with more certainty than has heretofore been present. Once the instrument has reached the ligamentum flavum ***46,*** portions of the ligamentum flavum ***46*** may be separated with a tissue separation device so as to relieve pressure on the spinal nerves ***42.*** If instrument comprises a tissue separation tool, instrument may be inserted into the ligamentum flavum ***46*** to separate portions of the ligamentum flavum ***46.*** However, if instrument comprises a cannula, instrument will be positioned adjacent the ligamentum flavum ***46*** in the region of interest and a tissue separation device ***100*** may be advanced through instrument toward ligamentum flavum ***46*** and inserted in ligamentum flavum ***46*** in the region of interest to retract tissue therefrom. In some embodiments, tissue separation can be performed generally from posterior to anterior across the interlaminar space ***38*** and then laterally along the anterior portion of the ligamentum flavum ***46*** if desired. The actual depth of the tip of instrument (or any tissue separation device ***100*** passing through instrument in the case instrument is a cannula) in the general direction of the sagittal ***54*** plane may be adjusted with guidance from the caudal-cranial posterior-lateral view and appropriate retraction/advancement of instrument and appropriate adjustment of instrument between about 5 and about 90 degrees relative to the posterior back surface ***70.***

### E. Tissue Separation

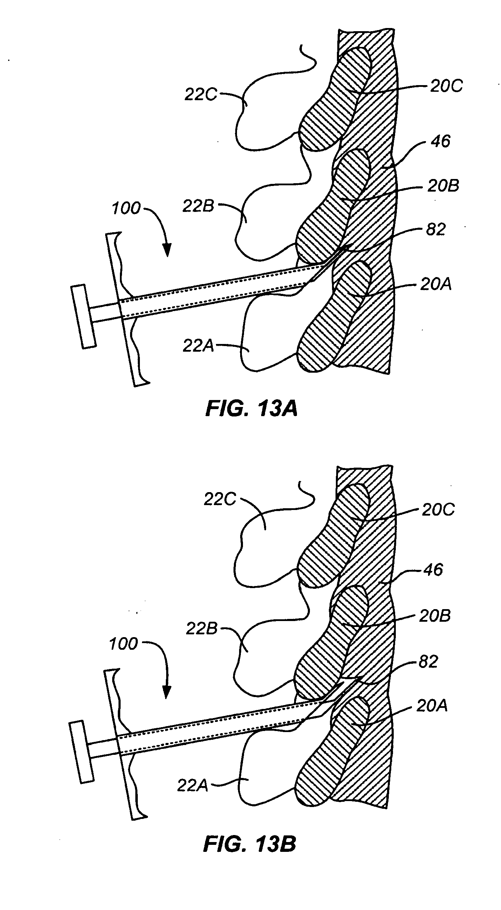

**FIGS. 13A-B** schematically illustrate the separation of a portion of tissue ***82*** by device ***100.*** In some embodiments, an instrument, such as a portal or cannula (not shown), may be employed to provide percutaneous access to tissue ***82.*** For instance, tissue separation device ***100*** may be inserted into and advanced through such a portal or cannula to reach targeted tissue ***82.*** U.S. Publication US 2007/0055263, discloses several tools, devices and methods for employing a portal to provide percutaneous access to a tissue of interest. If a portal or cannula is used to guide device ***100,*** device ***100*** may be passed through the cannula to reach the tissue of interest.

Regardless of the manner in which tissue separation device ***100*** reaches the tissue of interest (*e.g*., by portal or otherwise), device ***100*** is preferably advanced to the tissue of interest ***82*** without actuation of the tissue separation device ***100.*** Where the tissue separation device is actuated by an actuator, such as a motor, this would occur with the motor off (*i.e.,* without drive member ***132*** actuated).

**FIGS. 13A-B** schematically illustrate the separation of a portion of ligamentum flavum ***46*** by device ***100.*** In some embodiments, a portal or cannula (not shown) may be employed to provide percutaneous access to ligamentum flavum ***46.*** For instance, ligament separation device ***100*** may be inserted into and advanced through such a portal or cannula to reach ligamentum flavum ***46.*** U.S. Publication US 2007/0055263 discloses several tools, devices and methods for employing a portal to provide percutaneous access to a tissue of interest. If a portal or cannula is used to guide device ***100,*** device ***100*** may be passed through the cannula to reach the tissue of interest. As an exemplary embodiment, the method described below will refer to separation of the ligamentum flavum ***46*** from the lamina of the posterior aspect of the spine. Where the device ***100*** is passed through a cannula, to reduce the overall profile of the device, it may be desirable to form the upper separation member ***112*** and lower separation member ***122*** from a material that has shape memory qualities such that the members ***112, 122,*** can be extended (flattened) as the device is advanced through the cannula, and then assume its tissue separation configuration (shown in **FIGS. 6-9**) after the distal end of the device ***100*** exits the distal end of the cannula and is no longer constrained.

Upper and lower separation members ***112, 122*** are preferably inserted in a closed position as shown in **FIGS. 13A-B** between ligamentum flavum ***46*** and interior laminar edge. Device ***100*** may be inserted such that outer surface of upper member ***112*** contacts inner surface of lamina ***20*** while outer surface of lower member ***122*** contacts ligamentum flavum ***46.*** To separate ligamentum flavum ***46*** from lamina ***20,*** lower member ***122*** may be moved in a distal direction to force ligamentum flavum ***46*** from the lamina ***20.*** In embodiments with inner and outer handles, inner handle ***130*** may be pulled back in a proximal direction forcing upper member ***112*** in a proximal direction and apart from lower member ***122.*** Alternatively, outer handle ***136*** may be pushed in distal ***96*** direction forcing lower member ***122*** away from upper member ***112*** in a distal direction ***96*** as shown in **FIG. 13B****.** The force of pushing and/or pulling members ***112. 122*** apart causes separation of the ligamentum flavum ***46*** from lamina ***20.*** In further embodiments, instead of manually pulling and/or pushing handles as described above, device ***100*** may use any suitable means to move separation members ***112, 122*** apart such as a screw or threaded mechanism, ratcheting mechanism, actuator, or combinations thereof. Once the ligamentum flavum ***46*** and lamina ***20*** are sufficiently separated, a variety of suitable procedures may be performed on the now exposed surfaces of ligamentum flavum ***46*** and lamina ***20.***

Once separation of ligamentum flavum ***46*** and lamina ***20*** is no longer desired, upper and lower separation members ***112, 122*** may be drawn back together in a closed position and retracted from the body of the mammalian patient. Device ***100*** may be re-inserted and the separation techniques described above may be repeated to separate tissue, ligaments and/or bone in different parts of the mammalian body. Accordingly, although device ***100*** has been described with respect to the spine, device ***100*** may be used in other musculoskeletal parts of the body such as without limitation, knee, shoulder, ankle, etc.

The components of tissue separation device ***100*** (*e.g*., hollow body ***110,*** inner body ***120,*** separation members ***112, 122,*** etc.) may comprise any suitable material(s) including without limitation metals (*e.g*., stainless steel, titanium, etc.), non-metals (*e.g*., polymer, composites, etc.) or combinations thereof. The components of tissue separation device ***100*** are preferably manufactured from a durable biocompatible material such as titanium or stainless steel, but may alternatively be polymeric.

In addition, the components of tissue separation device ***100*** may be manufactured by any suitable methods. Examples of suitable methods include casting or molding, machining, laser cutting, EMD, or combinations thereof. In some embodiments, distal tip may be electro polished to for sharpening. The components of tissue separation device ***100*** may be assembled by any suitable method including without limitation welding, press fitting, or combinations thereof.

Once a user or surgeon has reached the desired hard or bony tissue, device ***100*** may be switched on. Once motor is actuated, drive pivot member ***132*** rotates tissue separation member ***102,*** thereby allowing the exposed portion of tissue separation member ***102*** to begin separating tissue at the target site. Distal end ***96*** of device ***100*** may be moved in a proximal and distal (*e.g*., back and forth) direction to facilitate separation of the tissue. Device ***100*** may have a switch to alter the direction at which tissue separation member ***102*** moves. In other words, motor may be switched to move or drive the tissue facing surface of tissue separation member ***102*** in a distal direction or a proximal direction.

In general, tissue ***82*** may be any type of tissue to be separated from another tissue within a mammalian patient including without limitation, soft tissue, fat, muscle, or bone. When used to treat spinal stenosis caused by a thickened ligamentum flavum ***46,*** distal end ***96*** of device ***100*** is preferably inserted into the stenotic ligamentum flavum ***46,*** preferably posterior to a safety zone ***80,*** in order to safely cut and remove portions of the thickened ligamentum flavum ***46*** (see **FIG. 13**), thereby reducing the stenosis.

Device ***100*** may also be used in conjunction with one or more other devices. The additional devices (not shown) may be used, for example, to first excise soft tissue such as ligaments or muscle and create a passageway to bone tissue. Device ***100*** may then be inserted into the passageway with the device ***100*** turned off (*e.g*., tissue separation member ***102*** not actuated).

The process of inserting device ***100*** into tissue, separating portions of tissue, and reinserting device ***100*** into the body may be repeated until the desired amount of tissue ***82*** has been separated or a sufficient space has been created. Referring briefly to **FIG. 14A,** when device ***100*** is employed to separate portions of thickened ligamentum flavum ***46,*** this process may be repeated until the spinal canal is adequately decompressed. Further, when device ***100*** is employed to separate portions of thickened ligamentum flavum ***46,*** distal end ***96*** of device ***100*** is preferably controlled to remain within ligamentum flavum ***46*** and not penetrate safety zone ***80.*** Nonetheless, safety zone ***80*** is preferably provided so that even an inadvertent penetration into epidural space ***44*** by device ***100*** will not result in damage to the dural sac ***48*** or nerves ***42.***

While embodiments of the invention have been shown and described, modifications thereof can be made by one skilled in the art without departing from the spirit and teachings of the invention. The embodiments described and the examples provided herein are exemplary only, and are not intended to be limiting. Many variations and modifications of the invention disclosed herein are possible and are within the scope of the invention. Accordingly, the scope of protection is not limited by the description set out above, but is only limited by the claims which follow, that scope including all equivalents of the subject matter of the claims.

While preferred embodiments of the present invention have been shown and described herein, it will be obvious to those skilled in the art that such embodiments are provided by way of example only. Numerous variations, changes, and substitutions will now occur to those skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A device for separating a first tissue from a second tissue at a surgical site, comprising:
a hollow body having a distal end and a proximal end, wherein the distal end further comprises an upper separation member extendable laterally from the hollow body at a first angle;
an inner member having a distal end and a proximal end and slidably disposed within the hollow body, wherein the distal end of the inner member has a lower separation member extendable laterally from the inner member at a second angle and wherein the upper separation member and the lower separation member have a first configuration and a second configuration.

2. The device of claim **1** wherein the first angle is greater than 90 degrees.

3. The device of claim 1 or claim **2** wherein the second angle is the same as the first angle.

4. The device of any one of the preceding claims wherein the hollow body and the inner member are substantially tubular.

5. The device of any one of the preceding claims wherein the upper separation member further comprises an outer face and an inner face and the lower separation member further comprises an outer face and an inner face and further wherein the separation members are constructed and arranged so that the inner face of the lower separation member is spaced between 3 and 16 mm from the inner face of the outer separation member in the first configuration.

6. The device of any one of the preceding claims further comprising a handle attachable to the proximal end of the hollow body.

7. The device of any one of the preceding claims wherein the upper separation member and the lower separation member have distal ends which are beveled.

8. The device of any one of claims **1** to 6 wherein the upper separation member and the lower separation member are angled to form a sharpened tip.

9. The device of any one of the preceding claims further comprising a means for moving the upper separation member and the lower separation member between the open configuration and the closed configuration, the means being located at the proximal ends of the hollow body and the inner member.

10. The device of any one of the preceding claims further comprising an actuator coupled to the inner member, which actuator may optionally be a motor, and which motor may optionally be disposed within the or a handle.

11. The device of any one of the preceding claims wherein either:
the first tissue and the second tissue are connective tissue; or
the first tissue is ligament and the second tissue is bone; or
the first tissue is a laminae of a vertebral body and the second tissue is a ligamentum flavum.

12. A kit for tissue separation comprising the device of any one of the preceding claims and
packaging.

13. The kit of claim **12** further comprising an injectable medium.

14. The kit of claim **13** wherein the injectable medium is a contrast medium, which contrast medium may optionally be a hydrophillic-lipophillic block copolymer gel.

15. The kit of any one of claims **12** to **14** further comprising a guide adaptable for use with the device.
